# EUROPEAN PATENT APPLICATION

(11) **EP 1 482 032 A1**
(43) Date of publication of application: **01.12.2004**
(21) Application number: 03705072.1
(22) Date of filing: 12.02.2003
(51) Int. Cl.: C12N 13/00, C12M 1/42

(54) **MOLECULE VIBRATOR**

(30) Priority: 12.02.2002 JP 2002077310
(71) Applicant: The Mollennium Laboratories, Nisshin-shi, Aichi 470-0125 (JP); Meiryo Technica Corporation, Ltd., Nagoya-shi, Aichi 461-8670 (JP)
(72) Inventor: KOIDE, Masafumi, Nagoya-shi, Aichi 468-0051 (JP); SAKAMOTO, Yoshitaka, Aichi-gun, Aichi 470-0155 (JP); MIZUNO, Takaji c/o MEIRYO TECHNICA CORPORATION,LTD, Nagoya-shi, Aichi 461-8670 (JP)
(74) Representative: Zinnecker, Armin, Dipl.-Ing.
(86) International application number: PCT/JP2003/001424
(87) International publication number: WO 2003/068953

(57) **Abstract**

A voltage generation circuit (**20**) is provided which generates a voltage. A voltage application circuit (**30**) is provided which has electrodes (**11, 12**) which are fed a respective voltage for causing creation of an instantaneous high electric field in a region of interest as a target such as a living body tissue or cell. The voltage application circuit (**30**) is high-impedanced for excitation of a free resonant circuit including the target. It is arranged such that the relative position of the target with respect to one of the electrodes (**11, 12**) is adjustable.

## Description

### TECHNICAL FIELD

This invention relates generally to molecule vibration apparatuses capable of causing vibration of a molecule such as a gene in the field of clinical medicine, experimental medicine, biology, zoology, or botany. The present invention relates more particularly to a molecule vibration apparatus for causing creation of an instantaneous high electric field.

### BACKGROUND ART

In the past, methods of introduction of a gene into cells have been classified broadly into: methods making use of a reagent (e.g., calcium phosphate method, dextran method, liposome method et cetera); introduction methods employing a physical means (e.g., electroporation method, gene gun method, microinjection method et cetera); and virus methods (e.g., adeno-virus method, retro-virus method et cetera).

In addition, as disclosed in WO 01-55294A1, there is an apparatus capable of introduction of a molecule such as a gene. In this introduction apparatus, an instantaneous high voltage is applied for causing creation of a high electric field. Such a type of gene introduction equipment makes utilization of free damped vibration inherent to a molecule which is excited by application of an instantaneous high voltage, whereby a foreign molecule is introduced into cells.

However, such a conventional introduction apparatus has the limit of efficiency because it merely causes creation of an instantaneous high electric field.

On the other hand, in the past, there has been an apparatus operable to give electrical stimulation, such as a high potential medical treatment apparatus. In such an electrical stimulation therapy, however, forced vibrations are applied to a living body, so that most cell constituent molecules do not exhibit their original vibration characteristic features. These molecules come to undergo apparatus-dependent, inappropriate micro-vibration.

In addition, in an energization therapy through an electric conductor, an electric current enters a cell. As the result of this, intracellular molecules are unevenly distributed rather than being vibrated and there occurs formation of a hole penetrating completely through the cell membrane. This therefore produces the problem that it becomes difficult to bring about the effect of cell function activation through an intracellular molecular reaction.

Bearing in mind the above-described drawbacks, the present invention was made. Accordingly, an object of the present invention is to achieve efficiency improvement and damage reduction.

In addition, another object of the present invention is application to the activation of the physiological functions of living beings, the enhancement of growth and regeneration capabilities, and the therapy of various diseases.

### DISCLOSURE OF INVENTION

A first invention is directed to a molecule vibration apparatus which comprises a voltage generation means capable of generation of voltages and an electric field creation means having a pair of electrodes, which are fed a voltage generated by the voltage generation means, for causing creation of an instantaneous high electric field in a region of interest as a target such as a living body tissue or cell, the electric field creation means being high-impedanced for excitation of a free resonant circuit including the target. And, a movable means is provided which makes the relative position of the target with respect to one of the pair of electrodes adjustable.

In the first invention, since there is made a change in the relative position of the target with respect to one of the electrodes, this equalizes an electric field which is applied to a group of cells, in the case where a culture dish having a large surface area is used, in the case where a large tissue piece is used, or in the case where a porous culture dish is used.

In addition, a second invention is directed to a molecule vibration apparatus which comprises a voltage generation means capable of generation of voltages and an electric field creation means having a pair of electrodes, which are fed a voltage generated by the voltage generation means, for causing creation of an instantaneous high electric field in a region of interest as a target such as a living body tissue or cell, the electric field creation means being high-impedanced for excitation of a free resonant circuit including the target. And, a gas spray means is provided which sprays an electrical discharge from one of the pair of electrodes with a gas so that the electrical discharge is flare-shaped.

In the second invention, an instantaneous electrical discharge is dispersed inside a three-dimensional space by the spraying of gas.

Furthermore, a third invention is directed to a molecule vibration apparatus which comprises a voltage generation means capable of generation of voltages and an electric field creation means having a pair of electrodes, which are fed a voltage generated by the voltage generation means, for causing creation of an instantaneous high electric field in a region of interest as a target such as a living body tissue or cell, the electric field creation means being high-impedanced for excitation of a free resonant circuit including the target. And, a magnetic body is provided in the vicinity of the target so that an electrical discharge from one of the pair of electrodes is flare-shaped.

In the third invention, since the magnetic body is disposed in the vicinity of the target, an electron flow and an ionization element flare excited by an instantaneous electric filed are situated within a magnetic field. As the result of this, the electron flow and so forth receive a stress orthogonal to the direction of movement of the electron or flare and are dispersed extensively in a cell distribution region.

A fourth invention according to any one of the first to third inventions is disclosed which is characterized in that the target is contained in an insulative dish and one of the electrodes associated with the insulative dish is an electrically conductive electrode.

In the fourth invention, since the insulative dish is provided, a cell development liquid is positioned near the neutral point of an electrical impedance, when viewed from the electrodes facing each other across the target.

A fifth invention according to any one of the first to third inventions is disclosed which is characterized in that a container in which to contain the target is formed of any one of inorganic porous material, natural polymer compound's porous material, and synthetic polymer compound's porous material.

In the fifth invention, extra electrical conducting property and uniformity (electron pumping) are added to the insulative molecular structure.

A sixth invention according to any one of the first to third inventions is disclosed which is characterized in that an adjustment means capable of adjusting a voltage applied from one of the electrodes is provided.

In the sixth invention, mutual adjustment in the phase or cycle at the time of excitation of the electrode is performed.

A seventh invention according to any one of the first to third inventions is disclosed which is characterized in that a container in which to contain the target is adjustable in internal pressure.

In the seventh invention, the strength of relative molecular vibration by resonant excitation is increased or decreased between the molecule and the cell.

An eighth invention is directed to a molecule vibration apparatus which comprises a voltage generation means capable of generation of voltages and an electric field creation means having a pair of electrodes, which are fed a voltage generated by the voltage generation means, for causing creation of an instantaneous high electric field in a region of interest as a target such as a living body tissue or cell. And, the electric field creation means is high-impedanced for excitation of a free resonant circuit including the target so that molecular vibrations occur.

In the eighth invention, the density of electrons grows exponentially in the vicinity of a lead-out region, and kinetic energy that is given to peripheral tissue cells increases.

A ninth invention according to any one of the first to third inventions and the eighth invention is disclosed which is characterized in that a crystal material is disposed between one of the electrodes and the target.

In the ninth invention, the characteristics of the living body electrical circuit including the cell and the foreign molecule are controlled adequately.

A tenth invention according to the eighth invention is disclosed which is characterized in that the electric field creation means is configured such that the target is damaged or destructed by application of a high electric field to the target.

In the tenth invention, by adjustment of the degree of concentration of the application of a high electric field to a local region and of the frequency of occurrence of such application, either cytoclasis is induced to local cells or to tissues in the body or apoptosis is induced.

### Effects of Invention

In accordance with the first invention, it is arranged such that the relative position of the target and the electrode is made changeable, which arrangement makes it possible to provide equalization of an electric field that is applied to a group of cells, in the case where a culture dish having a large surface area is used, in the case where a culture dish for large tissue pieces is used, or in the case where a porous culture dish is used. As the result of this, it is possible to achieve improvement in efficiency and to facilitate comparative experiments using great numbers of test samples. Besides, it is possible to achieve a reduction in damage.

Further, in accordance with the second invention, since an instantaneous electrical discharge is dispersed, by the spraying of gas, within a three-dimensional space, this makes it possible to introduce foreign molecules distributed around cells into the cells while avoiding cell damage due to the electrical discharge concentration.

In addition, since the electrical discharge is instantaneous and is intensive and intermittent, this makes it possible to realize irradiation of a plasma flare capable of selecting condition settings depending on the cells. As the result of this, it becomes possible to prevent the rise in temperature due to the generation of heat and to enhance the introduction of molecules into cells and the fusion of cells.

In addition, in accordance with the third invention, since the magnetic body is disposed in the vicinity of the target, an electron flow and an ionization element flare excited by an instantaneous electric filed are situated within a magnetic field. As the result of this, the electron flow and so forth receive a stress orthogonal to the direction of movement of the electron or flare and are dispersed extensively in a cell distribution region. Therefore, molecular vibrations are converted such that foreign molecules are easily introduced into cells and, in addition, are converted such that cell membranes are easily fused.

In accordance with the fourth invention, since the dish is formed of insulative material, a development liquid for a cell or the like is positioned near the neutral point of an electrical impedance, when viewed from the electrodes facing each other across the target. As the result of this, molecular vibrations are distributed equivalently from the base point in a positive and negative direction, thereby promoting the entrance of foreign molecules into cells.

In addition, in accordance with the fifth invention, since it is arranged such that a container in which to hold the target is formed of a porous material, this arrangement makes it possible to add extra electrical conducting property and uniformity (electron pumping) to the insulative molecular structure. Therefore, by the use of a container made of porous ceramic, it becomes possible to enhance the efficiency of gene introduction in comparison with the case where dishes made of plastic material are used, while avoiding the occurrence of damage to cells.

Furthermore, in accordance with the sixth invention, if it is arranged such that output voltage is adjusted, this arrangement makes it possible to perform mutual adjustment in the phase or cycle at the time of excitation of the electrode. As the result of this, it is possible to bring about a fluctuant variation (fuzzy control) in vibration characteristics, depending on the around-the-cell environment, the type of cell, or the type of foreign molecule. Stated another way, by bringing about variation control such as cycle variation of emphatic attenuation, it becomes possible to achieve high-efficiency molecule introduction without occurrence of damage.

In addition, in accordance with the seventh invention, it is arranged such that the internal pressure of a container in which to hold a target is adjusted. Such an arrangement makes it possible to enhance the introduction of molecules including genes by increasing or decreasing the strength of relative molecular vibration between a molecule and a cell by resonant excitation.

Furthermore, in accordance with the eighth invention, it is arranged such that a high electric field is discharged from one of the electrodes to its counter electrode through a predetermined region. As a result of such arrangement, the density of electrons grows exponentially in the vicinity of a lead-out region, and kinetic energy that is given to peripheral tissue cells increases. Consequently, while reducing the influence on other body regions, the effect on tissue cells of a damaged region of the body is enhanced sufficiently.

Additionally, in accordance with the ninth invention, it is arranged such that a crystal material is disposed between the electrode which generates a high potential and the target, which arrangement makes it possible to control in an adequate manner the characteristics of a living body electrical circuit including cells and foreign molecules, and to more strongly transmit vibrations by instantaneous stimulation to tissue cells.

Finally, in accordance with the tenth invention, by adjustment of the degree of concentration of the application of a high potential to a local region and the frequency of such application, either cytoclasis is induced to local cells or tissues in the body or apoptosis is induced.

### BRIEF DESCRIPTION OF DRAWINGS

Figure **1** is a top plan view showing a molecule vibration apparatus according to a first embodiment of the present invention;
Figure **2** is a top plan view showing a table of the first embodiment;
Figure **3** is a cross-sectional side view showing the molecule vibration apparatus of the first embodiment;
Figure **4** is a front view showing an electrode;
Figure **5** is a block diagram showing an electric system of the first embodiment;
Figure **6** is a flow diagram showing a voltage application procedure of the first embodiment;
Figure **7** is a top plan view showing a molecule vibration apparatus according to a second embodiment of the present invention;
Figure **8** is a side view showing the molecule vibration apparatus of the second embodiment;
Figure **9** is a front view showing the molecule vibration apparatus of the second embodiment;
Figure **10** is a front perspective illustration showing a part of the molecule vibration apparatus of the second embodiment;
Figure **11** is a system diagram showing a gas spray means of the second embodiment; and
Figure **12** is a perspective illustration showing a part of a molecule vibration apparatus according to an eighth embodiment of the present invention.

### BEST MODE FOR CARRYING OUT INVENTION

### Embodiment 1

Hereinafter, a first embodiment of the present invention will be described in detail with reference to the drawings.

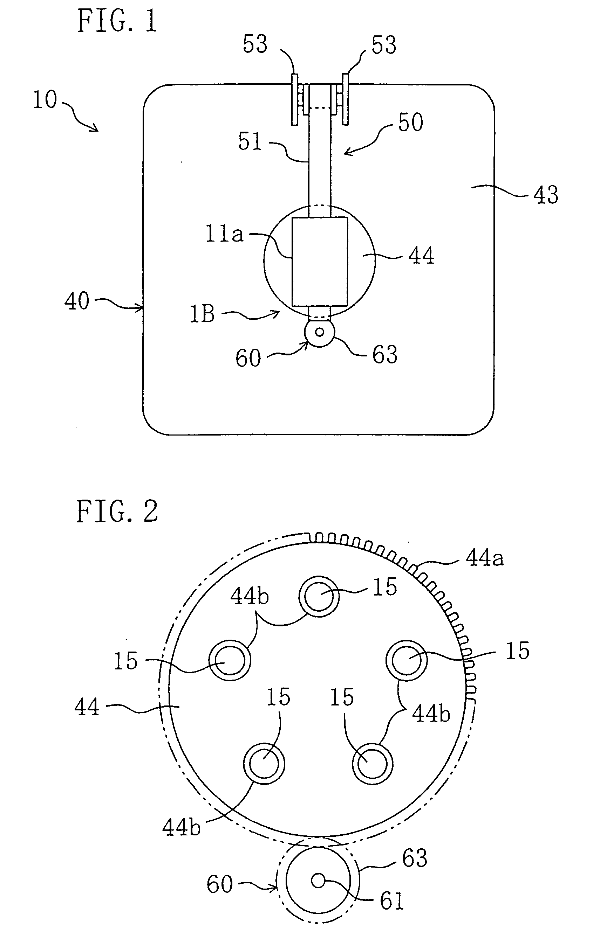

Referring to Figures **1-3**, there is illustrated a molecule vibration apparatus **10** of the present embodiment in the form of a gene introduction apparatus capable of introducing a gene which is a molecule into cells.

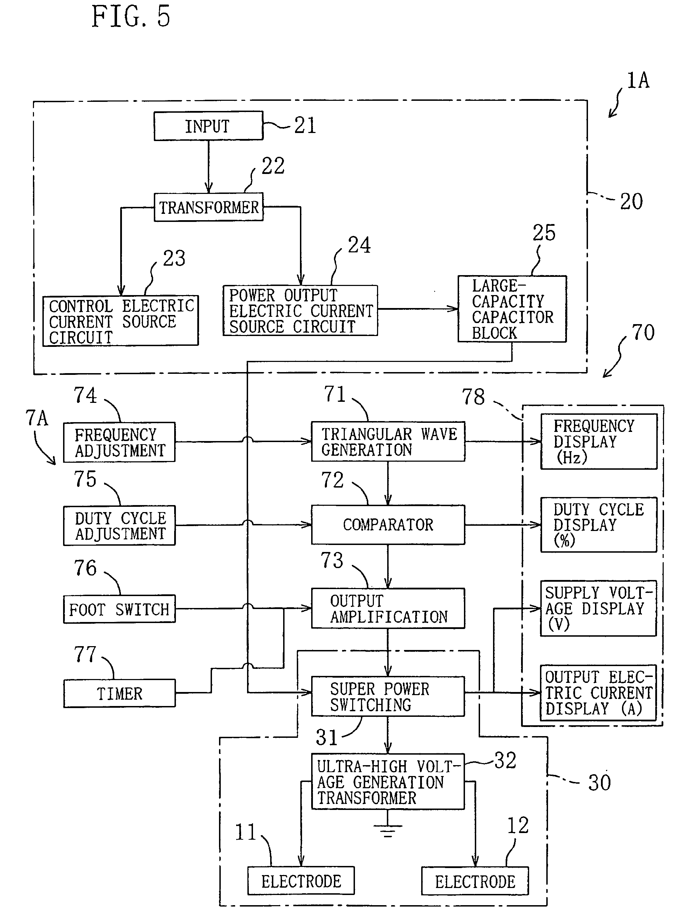

As shown in Figure **5,** the molecule vibration apparatus **10** is provided with an electric system **1A**. The molecule vibration apparatus **10** has a voltage generation circuit **20** and a voltage application circuit **30.**

The molecule vibration apparatus **10** is provided with a single pair of electrodes **11**, **12.** The molecule vibration apparatus **10** has a lower support platform **40** and an upper support platform **50**.

The lower support platform **40** is provided with a support plate **43** which is attached to a base plate 41 through support legs **42**. Formed on top of the support plate **43** is an electric mount part **43a** onto which a lower electric electrode **11** is mounted. A table **44** is mounted on top of the electrode mount part **43a**.

The table **44** is a rotatable turn table with external teeth **44a** formed on the periphery thereof. The table **44** is connected, through a connection means **60**, to a motor **45** mounted on the base plate **41**. The connection means **60** comprises a shaft **61** having, at its respective ends, a gear **62** and a gear **63**. And, the lower gear **62** is in engagement with the motor **45** while the upper gear **63** is in engagement with the external teeth **44a** of the table **44**.

Of the pair of the electrodes **11, 12**, the lower electrode **11** is attached to the electrode mount part **43a**. A fixed arm **46** is attached to an end of the electrode mount part **43a.** An electric power supply part **11a** is attached to the lower electrode **11,** and an electric discharge surface of the lower electrode **11** is exposed at the upper surface of the electrode mount part **43a**.

A movable arm **51** is attached to an electric power supply part **12a** to form the upper support platform **50.** The movable arm **51** is connected, through a pin **52**, to the fixed arm **46**, and the pin **52** is provided with a handle **53**. The movable arm **51**, when the handle **53** is rotated, rotates relative to the fixed arm **46** so that the electric power supply part **12a** moves vertically.

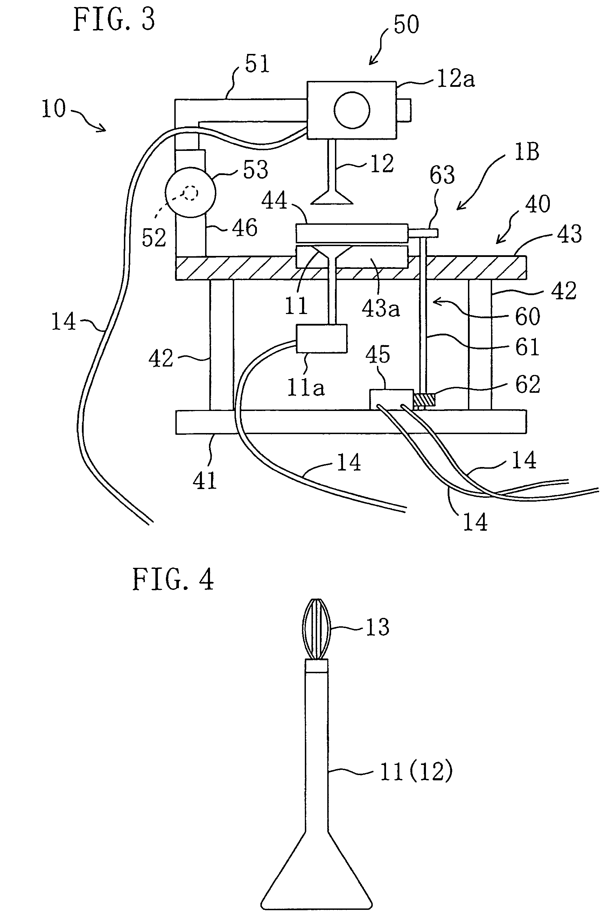

Of the pair of the electrodes **11, 12,** the upper electrode **12** is attached to the electric power supply part **12a**. The upper electrode **12** and the lower electrode **11** are disposed such that their electrical discharge surfaces face each other. As shown in Figure **4**, the upper electrode **12** and the lower electrode **11** are provided, at their ends, with a respective connection terminal **13**. And, the connection terminals **13** are connected to the electric power supply part **11a** and to the electric power supply part **12a**, respectively.

In addition, the table **44** and the connection means **60** constitute a moving means **1B** capable of making the relative position of the target and the electrodes **11, 12** movable.

The electric power supply parts **11a, 12a** and the motor **45** are each connected to a respective electric power cable **14**.

Referring to Figure **2**, a plurality of openings **44b** are formed in the table **44** and each opening **44b** houses therein a dish **15**. In other words, in the table **44** a plurality of dishes **15** are circumferentially arranged. And, each dish **15** contains cells and molecules being introduced into the cells, and these cells and molecules are targets. It is arranged such that each dish **15** lies intermittently between the pair of electrodes **11, 12**.

As shown in Figure **5,** the electric system **1A** has the voltage generation circuit **20** and the voltage application circuit **30**. The voltage generation circuit **20** constitutes a voltage generation means, and is provided with an alternate current input part **21**, a transformer **22**, a control electric current source circuit **23**, a power output electric current source circuit **24**, and a capacitor block **25**. The alternate current input part **21** is configured such that ac power is fed to the transformer **22**. The transformer **22** is configured such that it performs voltage transformation of alternate current power and outputs electric power of a predetermined voltage level to the control electric current source circuit **23** and to the power output electric current source circuit **24**. The power output electric current source circuit **24** is configured such that it outputs a predetermined high voltage to the capacitor block **25**.

The voltage application circuit **30** constitutes an electric field creation means for creating a high electric field between the upper electrode **12** and the lower electrode **11**, and is provided with a switching circuit **31** and a ultra-high voltage generation transformer **32**. The switching circuit **31** is configured such that it receives electric power outputted from the capacitor block **25** and supplies, in a given cycle, the ultra-high voltage generation transformer **32** with predetermined electric power. And, the upper electrode **12** and the lower electrode **11** are connected and the switching circuit **31** is turned off, and an instantaneous high voltage (high electric field) which is a single spike is generated on the secondary side of the ultra-high voltage generation transformer **32**. By virtue of generation of such a high voltage, a sine attenuation wave alternate voltage is induced.

Although not shown diagrammatically, the electric discharge surfaces of both the electrodes **11, 12** are covered with high insulating covering material. Stated another way, since the electrodes **11, 12** are coated with a high insulating covering material, this allows them to function as equivalent capacitors configured according to the dielectric constant.

The electrodes **11, 12** are so configured as to apply an instantaneous voltage of high level to a target including cells and molecules of interest. And, the voltage application circuit **30** constitutes an external resonant circuit including the target. When both the electrodes **11, 12** are energized, the voltage application circuit **30** operates as a differentiator with respect to the external resonant circuit including the target and supplies the external resonant circuit with a large trigger voltage.

A signal generation means **70** for generating switching signals is connected to the switching circuit **31.** The signal generating means **70** is provided with a triangular wave generation circuit **71,** a comparator **72,** and an output amplification circuit **73.**

Connected to the signal generation means **70** are a frequency adjustment circuit **74**, a duty cycle adjustment circuit **75**, a foot switch **76**, and a timer **77**. In addition, the signal generation means **70** is configured such that it outputs signals including a frequency signal to a display means **78** for displaying frequency information, supply voltage information, or other information.

The frequency adjustment circuit **74** and the duty cycle adjustment circuit **75** constitute an adjustment means **7A** for adjustment of the voltage applied to the electrodes **11, 12**. Furthermore, the adjustment means **7A** is configured such that it stores voltage-application conditions such as previous voltage-application conditions for making adjustment to a voltage condition or other condition corresponding to for example the target.

### Gene Introduction Method

Hereinbelow, a gene introduction method by the above-described molecule vibration apparatus **10** will be described.

In the first place, a dish **15,** previously loaded with cells and genes (molecules) being transferred to the cells, is set onto the table **44**. Thereafter, a precipitous high voltage is applied to the lower electrode **11** and to the upper electrode **12** and, as a result, the voltage application circuit **30** constitutes an external resonant circuit including a target composed of cells and genes. This external resonant circuit **30** including the target is excited in which an instantaneous voltage serves as a trigger voltage, and resonance occurs. Following the trigger voltage, a voltage variation in amplification modulation wave which is a sum of electron and charged molecule dynamic states is observed during the cell resonance. By application of stimulation to the target by such an output voltage, the circuit, whose center is a voltage application point, forms an electrically free resonant circuit, and a sinusoidal damped oscillation is induced. Both the cells and the genes are also incorporated into the free resonant circuit and vibrate in sync with the natural resonant characteristics.

As the result of this, a gene in the vicinity of the membrane of a cell, when given an electric field identical with that to which the gene itself is charged, moves towards the opposite electric field. On the other hand, a gene in the vicinity of the membrane of a cell, when given an electric field opposite to that to which the gene itself is charged, the gene moves towards an electric field capable of neutralization. And, as a result of continuous movement based on the natural resonant characteristics and repetition of reversible polarity inversion of the cell membrane, the gene exhibits an accelerative behavior. As the result of this, the gene passes through the cell membrane, comes into collision with a molecule within the cell, and is diffused, thereby becoming a part of the structure of a nuclear gene of a cell nucleus and showing a desired effect.

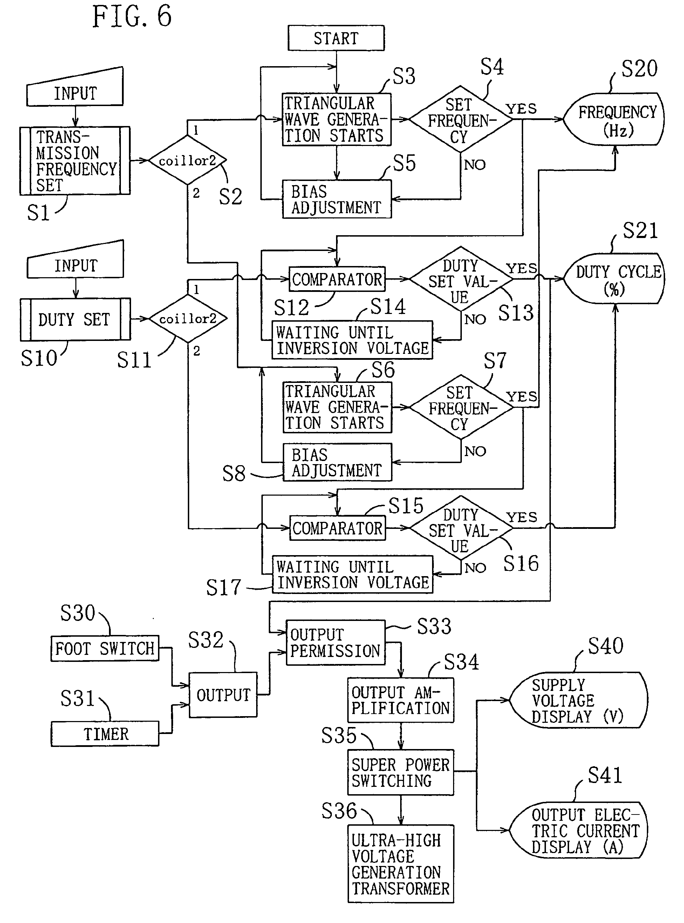

The above-mentioned voltage application operation will be described with reference to a flow diagram of Figure **6**.

In the first place, when a transmission frequency is set (see S1), generation of a triangular wave to the electrodes **11, 12** starts. Bias adjustment is continued until the frequency of the triangular wave becomes a set frequency (see S2-S5 and S6-S8).

On the other hand, when a duty is set (see S10), there is made a comparison between the set duty and the set frequency for the electrodes **11, 12** , and the duty is adjusted until it becomes a set value (see S11-S14 and S15-S17). In other words, by monitoring the rise and the fall of the frequency of the triangular wave, the duty is adjusted to a predetermined value.

The frequency and the duty cycle are displayed on a display means **78** (see S20 and S21).

In addition, when the start of output is permitted by either the foot switch **76** or the timer **77**, the output of the comparator **72** is amplified and the switching circuit **31** starts turning on or off. Then, the ultra-high voltage generation transformer **32** supplies both the electrodes **11, 12** with an instantaneous high voltage (see S30-36).

The supply voltage and the output electric current are displayed on the display means **78** (see S40 and S41).

On the other hand, since the table **44** rotates by the driving of the motor **45**, an instantaneous high voltage is applied intermittently to the plurality of dishes **15** upon application of an instantaneous high voltage between the electrodes **11, 12**.

Furthermore, the vertical position of the upper electrode **12** is adjusted by the operation of the handle **53**, whereby the distance between the upper electrode **12** and the lower electrode **11** is adjusted.

### Effects of First Embodiment

Therefore, in accordance with the first embodiment, it is arranged such that the table **44** rotates, which arrangement makes it possible to equalize an electric field which is applied to a group of cells in the case where culture dishes having a large surface area, culture dishes for large tissues, or porous culture dishes are used. As the result of this, it becomes possible to achieve improvement in efficiency and to facilitate comparative testing which uses large numbers of test bodies. Besides, it is possible to achieve damage reduction.

In addition to the above, for example, by voltage adjustment, it becomes possible to permit mutual adjustment in the phases or cycles of the electrodes **11, 12** at the time of excitation thereof. As the result of this, it is possible to cause fluctuant variation, such as fuzzy control, in vibration characteristics depending on the around-the-cell environment, the type of cell, or the type of foreign molecule. To sum up, by the controlling of variation such as cycle variation of emphatic attenuation, it becomes possible to achieve high-efficiency molecule introduction without occurrence of damage.

### Embodiment 2

Hereinafter, a second embodiment of the present invention will be described in detail with reference to the drawings.

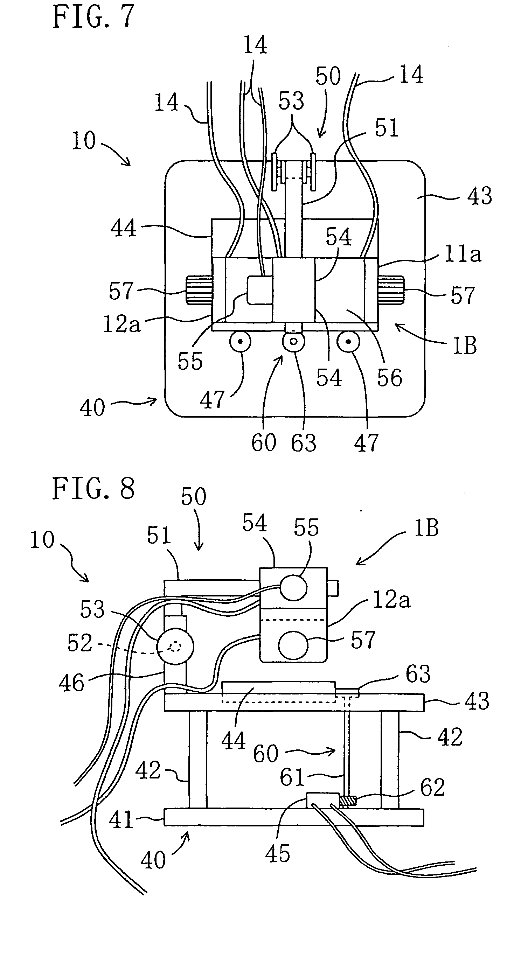

As shown in Figures 7 through 10, in the present embodiment it is arranged such that an electrical discharge between the electrodes **11, 12** is flare-shaped.

More specifically, the table **44** is mounted directly on top of the support plate **43** of the first embodiment. The table **44** is constructed such that dishes **15** are set thereon and is in engagement with the gear **63** of the connection means **60**. And, the table **44** is configured such that it is allowed to reciprocate in a lateral direction (X-axis direction) in Figure 7. The table **44** is guided by a guide roller **47**.

On the other hand, the upper support platform **50** is provided with a moving member **54** mounted on the movable arm **51**. The moving member **54** is equipped with a motor **55** and is mounted movably on the movable arm **5**1. More specifically, the moving member **54** is configured movably in a vertical direction (Y-axis direction) relative to the movable arm **51** in Figure **7**.

The table **44**, the connection means **60**, the moving member **54** et cetera together constitute a movable means **1B**.

The moving member **54** is provided with two electric power supply parts **11a, 12a** through a mount plate **56.** More specifically, the electric power supply parts **11a, 12a** are provided with needle-like electrodes **11, 12,** respectively, wherein an instantaneous high voltage is applied to the one pair of electrodes **11, 12.** The one pair of electrodes **11, 12** are disposed in a horizontal direction, and the electric power supply parts **11a, 12a** are provided with a respective handle **57** operable to adjust the distance between the electrodes **11, 12**.

In addition, the mount plate **56** is provided with a nozzle **81** of a gas spray means **80**. The gas spray means **80** sprays an electrical discharge from the electrodes **11, 12** with a gas so that the electric discharge is flare-shaped.

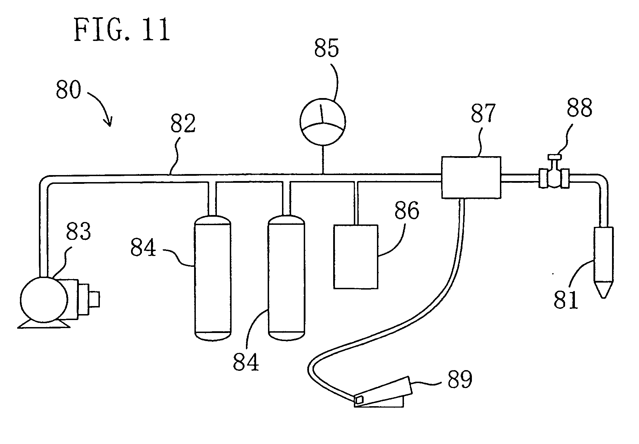

Referring to Figure **11**, the gas spray means **80** is comprised of a pipe **82** connected to the nozzle **81**, and a compressor **83** and gas tanks **84, 84** are connected to the pipe **82.** The compressor **83** and the gas tanks **84, 84** are connected to the pipe **82.** The pipe **82** is provided with a pressure gauge **85,** a pressure regulation tank **86,** an electromagnetic valve **87**, and a needle valve **88**, wherein, when the electromagnetic valve **87** is placed in the open position by a foot switch **89**, a gas is released from the nozzle **8**1.

The nozzle **81** is situated above the midway point between the one pair of electrodes **11, 12**. An electric discharge and an ion wind of ionization elements are expanded downwardly and a gas is sprayed so that this electrical discharge effect reaches a target contained in the dish **15**.

The gas sprayed from the nozzle **81** is composed of any one of air, carbon dioxide, argon, nitrogen, and helium or a mixed gas thereof.

Accordingly, when an instantaneous high voltage is applied to the one pair of electrodes **11, 12,** for example an arc electrical discharge occurs between the electrodes **11, 12**. At that time, a gas is sprayed from the nozzle **81**, so that the arc is converted into an instantaneous ion flare dispersed within a space, and cell groups and genes in the dish **15** as a culture dish are irradiated with the flare, along with an electric field variation. This causes molecules to vibrate and they are introduced into cells.

In addition, the dish **15** is shifted in a horizontal direction (X-axis direction) by the table 44 and the electrodes **11, 12** are shifted in a front-to-rear direction (Y-axis direction) by the moving member **54**, whereby the positional relationship between the dish **15** and the electrodes **11, 12** is adjusted.

As the result of the above, the strength of excitation of the external resonant circuit is integral-equalized, thereby achieving improvement in the molecule introduction efficiency.

Furthermore, since an instantaneous electrical discharge by resonance excitation is dispersed within a three-dimensional space by the spraying of a gas, this makes it possible to transfer foreign molecules distributed in the vicinity of a cell into the cell while avoiding cell damage due to the concentration of instantaneous electrical discharges.

In addition, since the electrical discharge is instantaneous and is intensive and intermittent, this makes it possible to realize irradiation of a plasma flare capable of selecting a condition set depending on the cells. This therefore makes it possible to prevent a rise in temperature due to the generation of heat which is a problem of the conventional plasma torch method, and to enhance the introduction of molecules into cells and the cell fusion.

Other configurations and operation/working-effects of the present embodiment are the same as the first embodiment. As a modification example of the present embodiment, it may be arranged such that the table **44** and so on are not movable. To sum up, in the present embodiment, it may be arranged such that only the gas spray means **80** is provided.

### Embodiment 3

Hereinafter, a third embodiment of the present invention will be described in detail.

In the present embodiment, for example, a magnetic body is disposed in the vicinity of the dish **15**.

For example, a neodymium magnet of the ring type is arranged in the vicinity of the underside surface of the dish **15** of the first embodiment. As the result of this, an ion flare over the dish **15** expands in a horizontal direction.

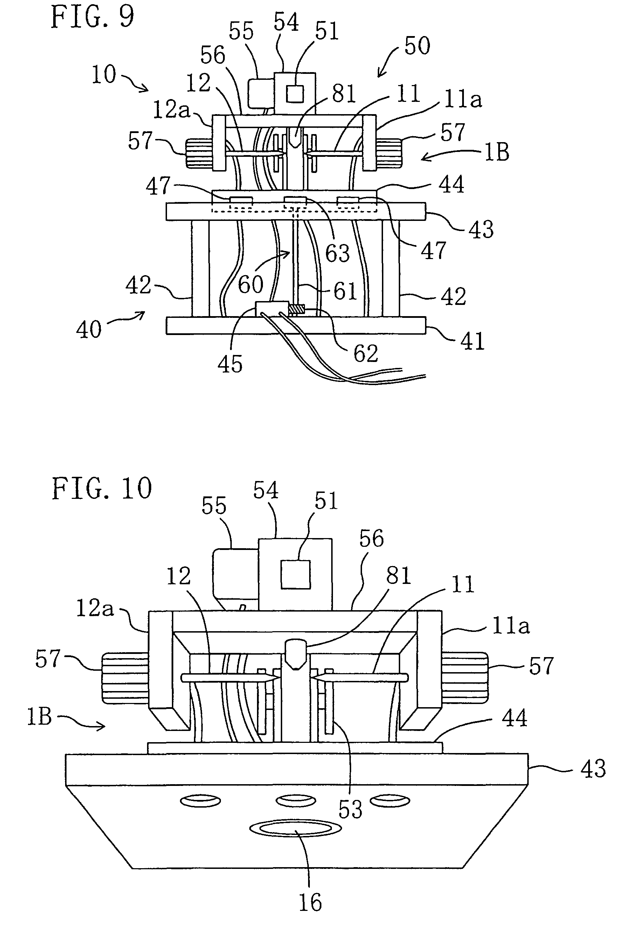

Alternatively, a neodymium magnet of the ring type is arranged in the vicinity of the underside surface of the dish **15** of the second embodiment. As the result of such arrangement, a flare distribution over the dish **15** expands in a horizontal direction. In other words, as shown in Figure **10**, a magnet **16** is attached to the support plate **43.**

That is, a stress, which attracts an electric current in a direction orthogonal to both the magnetic field direction and the electric current direction, acts on an electric current present in a magnetic field by Fleming's law. Accordingly, a current of electrons and an ionization element flare excited by an instantaneous electric field receive, by being situated within a magnetic field, a stress orthogonal to the direction in which the electron or flare advances, and are dispersed with expanse.

Therefore, molecule vibrations are converted so that foreign molecules are easily introduced into cells, and that cell membranes fuse easily.

Accordingly, in the case where the magnetic body is implemented by a magnet, the supply of electric power to a coil is controlled for manipulation of the characteristics of an electromagnet, whereby it is possible to secondarily adjust the movement of ions and electrons forming an electric discharge or an arc flare by resonance excitation.

In addition, in the case where the magnetic body is implemented by a paramagnet, the magnet body can be shaped like a doughnut or a disk.

Furthermore, it is possible to perform secondary distribution control of an instantaneous electric field or an ion plasma by magnet position control, movement, or vibration.

Additionally, if a magnetic body is used as a material to form the electrodes **11, 12,** this makes it possible to control the distribution of electrons and ionization elements.

It may be arranged as follows. That is, a permanent magnet, which is a magnetostatic field generation source capable of stably supply of a constant magnetic field, serves as a bias magnetic field, and such a permanent magnet is combined with a controllable magnetic field generation source. In this case, it is possible to generate magnetic force lens effects, thereby making it possible to carry out efficient introduction of molecules into a fine or extensive target.

Other configurations and operation/working-effects of the present embodiment are the same as the first embodiment. As a modification example of the present embodiment, it may be arranged such that the table **44** and so on are not movable. To sum up, in the present embodiment, it may be arranged such that only a magnetic body such as the magnet **16** is provided.

### Embodiment 4

Hereinafter, a fourth embodiment of the present invention will be described in detail.

In the present embodiment, the dish **15** is formed of insulating material. As a result of such arrangement, for example, the lower electrode **11** of the first embodiment no longer need provision of an insulating body (not shown) and the electrodes **11, 12** are electrically conductive.

For example, if an electrically conductive polymer plate is applied to the lower electrode **11** of the first embodiment, the brightness of blue nitrogen plasmas to the insulative dish **15** containing cells increases further, thereby making it possible to improve the efficiency of introduction of genes into cell groups.

More specifically, in the case where cell groups and tissues into which molecules are introduced are contained in an insulative culture dish, insulation impedance becomes great between the electrodes **11, 12** on the side where there is a culture dish depending on the presence or absence of the interposition of a culture dish. When, at this time, trying to apply a sufficient ultra high voltage, a short-circuit electrical discharge tends to occur on the side where there is no interposition of a culture dish, thereby producing the problem that such a short-circuit electrical discharge causes damage to the cells.

To cope with such a problem, it is preferable to employ a metal-containing inorganic substance, an electrically conductive substance such as electrically conductive polymer, or the electrodes 11, 12 of low insulative property, on the side where an insulative culture dish is interposed between the cells and the electrodes **11, 12.**

If the low-insulation electrodes **11, 12** are employed on the side of a culture dish of high insulative property, this causes a cell expansion liquid to be poisoned near the neutral point of an electrical impedance, when viewed from the electrodes **11, 12** facing each other across the cells. Consequently, molecule vibrations are distributed from the base point equivalently in a positive and negative direction, thereby promoting the entrance of foreign molecules into the cells.

Other configurations and operation/working-effects of the present embodiment are the same as the first or second embodiment.

### Embodiment 5

Hereinafter, a fifth embodiment of the present invention will be described in detail.

In the present embodiment, the dish **15** is made of porous ceramic. Cell groups and foreign genes were placed in the porous ceramic dish **15**, and the effect of introduction of genes was examined in the introduction and manifestation of GFP genes encoding a jellyfish green fluorescent enzyme in CHO cells.

CHO cells were cultured in porous ceramic dishes, and GFP genes were introduced by application of resonance excitation caused by an instantaneous electric field. After an elapse of 24 hours, the level of manifestation of the GFP genes was confirmed by means of a fluorescence microscope. The use of porous ceramic dishes proved that the efficiency of gene introduction/manifestation was improved from three to four times, in comparison with the use of plastic dishes under the same conditions.

The insulation properties of various materials are defined by difficulty in electron movement and by dielectric constant. General ceramic and organic polymers are insulative; however, water vapor and ionization elements are contained in the atmosphere. Because of this, the transfer of electrons becomes easy in the air, and the air brings about insulation dissociation and exhibits conductive properties.

In view of the above, it is possible to add extra electrical conducting property and uniformity (electron pumping) to an insulative molecular structure by making such an arrangement that the insulating material of the electrodes **11, 12** and the dish **15** is porous in a molecule introduction of the resonance excitation type. Therefore, by use of a container made of porous ceramic, it becomes possible to enhance the efficiency of gene introduction in comparison with the case where the dish **15** is formed of plastic material, while avoiding the occurrence of damage to cells.

Other configurations and operation/working-effects of the present embodiment are the same as the first or second embodiment. In addition, the aforesaid porous material may be a porous material formed of a natural polymer compound or a porous material formed of a synthetic polymer compound, in addition to a porous material formed of an inorganic substance.

### Embodiment 6

Hereinafter, a sixth embodiment of the present invention will be described in detail.

Instead of using the dish 15, the present embodiment employs a container which contains cells and foreign molecules and which is adjustable in its internal pressure.

The container of the present embodiment is formed, for example, into a capsule, and is filled with an electrically conductive gel. When a high voltage is applied to the container, stimulation by an instantaneous electric field passes uniformly through the inside of the electrically conductive gel and is distributed evenly in a cell caught in the electrically conductive gel, thereby preventing the occurrence of damage to issues.

If the container is adjustable in internal pressure and the container is filled with gas such as air, liquid, gel, sol, or solid substance, this causes the impedance characteristic between the electrodes **11, 12** or between the electrodes **11, 12** and the cell to vary greatly.

Consequently, it is possible to improve the introduction of molecules including genes by increasing and decreasing the strength of relative molecular vibration by resonance excitation between a foreign molecule and a cell constituting molecule.

Other configurations and operation/working-effects of the present embodiment are the same as the first or second embodiment.

### Embodiment 8

Hereinafter, a seventh embodiment of the present invention will be described in detail.

The present embodiment is intended for seedlings or the like. More specifically, stimulation by an instantaneous electric field was applied, for 30 minutes a day, to saplings of the coffee tree under indoor cultivation. By such stimulation, the acceleration of molecular transition inside and outside the cells and the free resonance vibration of cell constituting molecule groups were brought about. As the result of this, in comparison with the case where no stimulation was applied, growth in sapling height was promoted obviously for a period of three days and, as a result of the activation in photosynthesis, growth of more lively, dark-green leaves was observed.

In addition, primary culture cardiac muscle cells and COS7 cells under cultivation in an atmosphere of carbon dioxide-containing saturated steam of low oxygen and 37 °C, were extracted in a germfree environment and were subjected to stimulation by an instantaneous electric field for five minutes. In this case, a greater increase in the number of cultured cells was observed after an elapse of 24 hours in comparison with the case where no stimulation was applied. Accordingly, such a promotion of cell growth will contribute to the regeneration medicine or to the mass production of useful cells.

### Embodiment 8

Hereinafter, an eighth embodiment of the present invention will be described in detail with reference to the drawings.

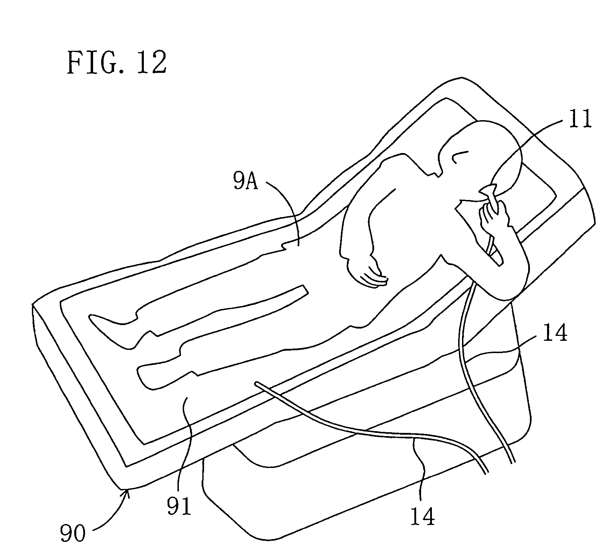

The molecule vibration apparatus **10** of the present embodiment is a medical treatment apparatus intended for blood flow insufficiency, neuroparalysis et cetera, as shown in Figure **12.**

More specifically, the molecule vibration apparatus **10** is configured for application to a person **9A** who is under medical treatment on a bed **90**. The bed **90** is electrically insulated from the floor. An electrically conductive mat **91** is provided on top of the bed **90**. This electrically conductive mate **91** constitutes a single electrode, e.g., the upper electrode **12** of the first embodiment. And, the subject **9A** lies face up on the electrically conductive mat **91**.

In addition, the subject **9A** presses one of the electrodes, i.e., the electrode **11**, against a specified body region which is a target for treatment and applies an instantaneous high voltage thereto. In other words, the electrode **11** is in the form of a hand piece which is hand-grippable by the subject **9A,** and the voltage application circuit **30** is configured such that it causes molecules in the target body region to vibrate.

Other configurations and operation/working-effects of the present embodiment are the same as the first or second embodiment. But neither the lower support platform **40** nor the upper support platform **50** is provided in the present embodiment.

The reason for the construction of the above-mentioned bed **90** is as follows. If the subject **9A** is placed in an electrically floating state without being connected to ground, electrons relatively evenly move into the air from the entire body or vice versa. Because of this, the density of kinetic electrons is low in a body region requiring a medical treatment. As the result of this, even when great instantaneous vibrations are applied, the kinetic energy that is applied to tissue cells at a body region to be treated will not reach satisfactory levels.

To cope with this, in the present embodiment it is arranged such that a high electric field is discharged from an electrode (which is the electrically conductive mat **91**) to the counter electrode **11**, through an arbitrary region of the subject's body. In this case, the density of electrons grows exponentially in the vicinity of a lead-out region, and kinetic energy that is given to peripheral tissue cells increases. Consequently, while reducing the influence on other regions of the subject's body, the effect on tissue cells of a damaged region of the subject's body is enhanced sufficiently.

For example, an instantaneous high electrical potential of 300,000 volts is applied so that an instantaneous high electrical potential is given to the subject **9A** from the electrically conductive mat **91** serving as an electrode. When the subject **9A** presses the electrode **11** against his/her body area requiring treatment, the kinetic energy of electrons given to the subject's entire body enters and leaves the electrode **11** from near the treatment-requiring body area.

Such therapy was performed for three months every other day for 15 minutes at one time. For the case of chronic facial nerve palsy, improvements in facial configuration due to neuroparalysis were observed and it became possible for the subject to do whistle exercises. And, the evaluation of the severity of neuroparalysis by scoring significantly improved from 32/40 to 36/40.

In addition, for the case of patients with lower limb blood flow insufficiency due to arteriosclerosis obliterans, by the application of stimulation by an instantaneous high electric field for 25 minutes at one time, the subject slightly felt vibrations like an electric shock at his/her limb ends. As just described, improvements in the peripheral blood flow were observed and a rise in the lower limb skin temperature were observed.

Furthermore, for the case of patients with facial nerve palsy, a cell molecule vibration therapy by resonance excitation was performed for one week for 25 minutes per day. This therapy achieved a reduction in facial lateral difference, thereby achieving obvious improvements in face configuration.

For the case of patients with chronic gastroenteritis, their abdomen enlarged feeling faded away and improvements in appetite were observed by execution of resonance excitation for 25 minutes at one time. Other than that, therapeutic effects, such as improvement in chronic headache, owing to the execution of molecular vibration of the resonance excitation type were observed for various pathologies.

In addition, the molecule vibration apparatus **10** of the present embodiment promotes transition of extracellular molecules into cells by stimulation by an instantaneous electric field. Besides, the molecule vibration apparatus **10** activizes the interaction between cell constituting molecules and the traffic of intracellular molecules by free resonance vibration.

Accordingly, cell physiological functions, such as the taking-in of nutritional elements or growth promoting elements by bio cells, the promotion of metabolic decomposition product discharge, the promotion of intracellular metabolic turnover, and other function, are activized. As the result of this, the growth of cells in medical treatments for injuries or morbid tissues and in regeneration medicine or the promotion effect of division of animal/plant cells including embryonal stem cells (ES cells) and cloned body cells becomes manifest. This contributes enormously to the improvement in medical treatment effect, development of new medical treatments, application to biologic craft, industrial efficiency in agriculture, forestry and fisheries, and resource saving.

Furthermore, conventional potential treatment apparatuses and so on are those which apply forced vibrations to a living body. Accordingly, most of the cell constituting molecules fail to render their original vibration characteristics, and occurrence of micro vibrations inappropriate for the cell physiological functions is inevitable. Tissue stimulation by existing dc conducting treatment equipment achieves nothing but results in cell membrane depolarization, therefore being unable to bring out an action of causing intracellular molecules to vibrate.

On the other hand, repetitive application of an instantaneous electric filed to the human body or to a local region of the human body brings free-resonating molecular movement to injured cells. Accordingly, for various disorders and injuries, the normalization of damage or morbid tissues, the functional restoration for morbid cells, the growth of remaining normal cells, or the differential growth power of stem cells which may slightly exist is induced.

To sum up, it is impossible for conventional methods to bring out an action which causes intracellular molecules to vibrate. Contrary to this, in accordance with the present embodiment, molecular motion is given to a local tissue, and strong molecule vibrations are excited in a target such as a damaged region and a morbid tissue. As a result, improvements in cell physiological function through the free vibration of cell constituting molecules and a variety of treatment effects by growth acceleration become manifest.

### Other Embodiments

In the eighth embodiment, the single electrode **11** and the electrically conductive mat **91** are provided. It however may be arranged such that a plurality of electrodes are provided.

Further, in the eighth embodiment, it may be arranged such that a crystal material is interposed between the terminal which generates a high potential (i.e., the electrically conductive mat **91**) and the body.

The crystal material is for example lithium tantalate, titanium oxide, silicon, quartz, sapphire, or diamond. These materials have inherent resonance properties and dielectric properties.

Depending on the angle of incidence, the resistance to the passage of photons and electrons is far small and coherent. Accordingly, if a crystal material is disposed between an output terminal and a living body or the like, this achieves adequate control of the characteristics of a living body electric circuit including cells and foreign molecules, as a result of which vibrations by application of instantaneous stimulation are more powerfully transmitted to cell tissues.

In addition, use of the crystal material may be applied to the first or second embodiment. For example, in the first or second embodiment it may be arranged such that a crystal material is disposed between the target and the electrode **11**. More specifically, the dish **15** may be formed of a crystal material.

For example, instantaneous high potential stimulation was applied to heart origin fibroblasts of the mouse on a culture dish made of lithium tantalate, quartz crystal, or crystallized glass. In this case, an increase in space beat sound and generation of a high tone sound inherent to the crystal were observed and the efficiency of introduction and manifestation of GFP genes was improved.

Furthermore, in the eighth embodiment the voltage application circuit **30** may be configured such that it damages or destroys a target.

To sum up, excision, irradiation, chemical therapy, or gene therapy for malignant tumors is performed. By adjusting the degree of concentration of the application of a high potential to a local region of the body and the frequency of application of such a high potential, either cytoclasis is induced to local cells or to body tissues or apoptosis is induced.

Especially, discharge energies from a plurality of application electrodes are vector-added together, so that other regions except for a target region are rendered harmless. At that time, it is preferable to provide vibrational energy enough for cell destruction to a specified local region or to bring about heat generation.

For example, an instantaneous high potential was applied to heart origin fibroblasts of the mouse cultured on a plastic dish at a level of 500,000 volts at a frequency of 100 times per second for 60 seconds.

As the result of the above, a slight rise in temperature of the culture dish was observed in a region of high electric field strength, and many blebs projected on the periphery of the region from the cells, after which cell apoptosis was induced. At this time, in adjacent regions of low electric field strength, no influence was observed on the cell shape or on the viability. This shows that the application of an instantaneous high potential brings about strong torsional motion to cell membranes, thereby bringing about an irreversible structural change to the lipid bilayer structure.

### INDUSTRIAL APPLICABILITY

As has been described above, the present invention provides a molecule vibration apparatus usefully applicable for the introducing of a molecule into cells. More specifically, the molecule vibration apparatus of the present invention is suitable for use in the gene therapy, regeneration treatment, introduction of medicinal substances into a target tissue or target cell, improvement in varieties of plants and animals, preparation of genetically-modified living being, cell fusion, and artificial material synthesis.

## Claims

1. A molecule vibration apparatus, comprising:
voltage generation means capable of generation of voltages, and
electric field creation means having a pair of electrodes, which are fed a voltage generated by said voltage generation means, for causing creation of an instantaneous high electric field in a region of interest as a target such as a living body tissue or cell, said electric field creation means being high-impedanced for excitation of a free resonant circuit including said target,
wherein movable means is provided which makes the relative position of said target and one of said pair of electrodes adjustable.

2. A molecule vibration apparatus, comprising:
voltage generation means capable of generation of voltages, and
electric field creation means having a pair of electrodes, which are fed a voltage generated by said voltage generation means, for causing creation of an instantaneous high electric field in a region of interest as a target such as a living body tissue or cell, said electric field creation means being high-impedanced for excitation of a free resonant circuit including said target,
wherein gas spray means is provided which sprays an electrical discharge from one of said pair of electrodes with a gas so that said electrical discharge is flare-shaped.

3. A molecule vibration apparatus, comprising:
voltage generation means capable of generation of voltages, and
electric field creation means having a pair of electrodes, which are fed a voltage generated by said voltage generation means, for causing creation of an instantaneous high electric field in a region of interest as a target such as a living body tissue or cell, said electric field creation means being high-impedanced for excitation of a free resonant circuit including said target,
wherein a magnetic body is provided in the vicinity of said target so that an electrical discharge from one of said pair of electrodes is flare-shaped.

4. The molecule vibration apparatus of any one of claims 1-3, wherein:
said target is contained in an insulative dish, and
one of said pair of electrodes associated with said insulative dish is an electrically conductive electrode.

5. The molecule vibration apparatus of any one of claims 1-3, wherein a container in which to contain said target is formed of any one of inorganic porous material, natural polymer compound's porous material, and synthetic polymer compound's porous material.

6. The molecule vibration apparatus of any one of claims 1-3, further comprising adjustment means capable of adjusting a voltage applied from one of said pair of electrodes.

7. The molecule vibration apparatus of any one of claims 1-3, wherein a container in which to contain said target is adjustable in internal pressure.

8. A molecule vibration apparatus, comprising:
voltage generation means capable of generation of voltages, and
electric field creation means having a pair of electrodes, which are fed a voltage generated by said voltage generation means, for causing creation of an instantaneous high electric field in a region of interest as a target such as a living body tissue or cell,
wherein said electric field creation means is high-impedanced for excitation of a free resonant circuit including said target so that molecular vibrations occur.

9. The molecule vibration apparatus of any one of claims 1-3 and 8, wherein a crystal material is disposed between one of said pair of electrodes and said target.

10. The molecule vibration apparatus of claim 8, wherein said electric field creation means is configured such that said target is damaged or destructed by application of a high electric field to said target.
